# EUROPEAN PATENT SPECIFICATION

(11) **EP 3 104 844 B1**
(45) Date of publication and mention of the grant of the patent: **12.02.2020**
(21) Application number: 15710913.3
(22) Date of filing: 13.02.2015
(51) Int. Cl.: A61K 9/16, A61K 47/10, A61K 47/20

(54) **COMPLEXES OF SIROLIMUS AND ITS DERIVATIVES, PROCESS FOR THE PREPARATION THEREOF AND PHARMACEUTICAL COMPOSITIONS CONTAINING THEM**
KOMPLEXE AUS SIROLIMUS UND DESSEN DERIVATEN, VERFAHREN ZUR HERSTELLUNG DAVON UND PHARMAZEUTISCHE ZUSAMMENSETZUNGEN DAMIT
COMPLEXES DE SIROLIMUS ET LEUR DÉRIVÉS, LEUR PROCÉDÉ DE PRÉPARATION ET COMPOSITIONS PHARMACEUTIQUES LES CONTENANT

(30) Priority: 14.02.2014 HU P1400075
(43) Date of publication of application: 21.12.2016
(73) Proprietor: Druggability Technologies IP Holdco Limited, Swatar, BKR 4013 (MT)
(72) Inventor: ANGI, Erzsébet Réka, 2094 Nagykovácsi (HU); SOLYMOSI, Tamás, 1139 Budapest (HU); KÁRPÁTI, Richard Balázs, 2800 Tatabánya (HU); FENYVESI, Zsófia, 1131 Budapest (HU); ÖTVÖS, Zsolt, 6640 Csongrád (HU); MOLNÁR, László, 2051 Biatorbágy (HU); GLAVINAS, Hristos, 6721 Szeged (HU); FILIPCSEI, Genovéva, 2151 Fót (HU); FERENCZI, Katalin, 1074 Budapest (HU); HELTOVICS, Gábor, 1122 Budapest (HU)
(74) Representative: Molnar, Istvan
(86) International application number: PCT/IB2015/051086
(87) International publication number: WO 2015/121836

(56) References cited:
- EP-A1- 1 759 724
- EP-A1- 1 952 807
- WO-A1-2006/094507
- US-A1- 2013 039 951
- US-A1- 2013 150 397

## Description

### FIELD OF THE INVENTION

The invention is directed to a stable complex with controlled particle size, increased apparent solubility and increased dissolution rate comprising as active compound Sirolimus or salts thereof, which is useful in the prophylaxis of organ rejection in patients receiving renal transplants, in the treatment of psoriasis, facial angiofibromas associated with tuberous sclerosis, fibrofolliculomas found in Birt-Hogg-Dubé Syndrome, chronic erosive oral lichen planus, Early Stage Cutaneous T-cell Lymphoma, Autoimmune Active Anterior Uveitis, dry eye syndrome, age-related macular degeneration, diabetic macular edema, noninfectious uveitis, telangiectasia, inflammatory skin diseases (dermatitis, including psoriasis and lichen ruber planus), Pachyonychia Congenita and in the suppression of angiogenesis pathways. More specifically, the complex of the present invention possesses increased apparent solubility, permeability and enhanced biological performance including significantly improved exposure, earlier tₘₐₓ, higher Cₘₐₓ and higher trough concentrations at 24 hours which will allow the reduction of the dose. Furthermore, the complex of the present invention possesses exceptional stability as a redispersed solution allowing the development of liquid based formulation for transdermal and other topical applications. The invention also relates to methods of formulating and manufacturing complex according to the invention, pharmaceutical compositions containing it, its uses in treatment using the complex and its compositions.

### BACKGROUND OF THE INVENTION

Sirolimus is a macrocyclic lactone produced by *Streptomyces hygroscopicus.* The chemical name of Sirolimus (also known as Rapamycin) is (3S,6R,7E,9R,10R,12R,14S,15E,17E,19E,21S,23S,26R,27R,34aS)9,10,12,13,14,21,22,23,24, 25,26,27,32,33,34, 34a-hexadecahydro-9,27-dihydroxy-3-[(1R)-2[(1S,3R,4R)-4-hydroxy-3-methoxycyclohexyl]-1-methylethyl]-10,21-dimethoxy6,8,12,14,20,26-hexamethyl-23,27-epoxy-3H-pyrido[2,1-c][1,4] oxaazacyclohentriacontine1,5,11,28,29 (4H,6H,31H)-pentone. Its molecular formula is C₅₁H₇₉NO₁₃ and its molecular weight is 914.2 g/mol. The structural formula of Sirolimus is illustrated as follows.

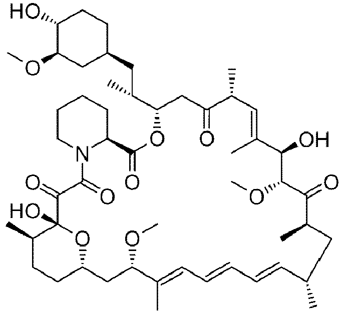

Sirolimus is a white to off-white powder and is insoluble in water, but freely soluble in benzyl alcohol, chloroform, acetone, and acetonitrile.

Rapamune is available for administration as an oral solution containing 1 mg/mL Sirolimus. Rapamune is also available as a tan, triangular-shaped tablet containing 0.5 mg Sirolimus, as a white, triangular-shaped tablet containing 1 mg Sirolimus, and as a yellow-to-beige triangular-shaped tablet containing 2 mg Sirolimus.

The excipients in Rapamune Oral Solution are Phosal 50 PG® (phosphatidylcholine, propylene glycol, mono- and di-glycerides, ethanol, soy fatty acids, and ascorbyl palmitate) and polysorbate 80. Rapamune Oral Solution contains 1.5% - 2.5% ethanol.

The excipients in Rapamune Tablets include sucrose, lactose, polyethylene glycol 8000, calcium sulfate, microcrystalline cellulose, pharmaceutical glaze, talc, titanium dioxide, magnesium stearate, povidone, poloxamer 188, polyethylene glycol 20,000, glyceryl monooleate, carnauba wax, dl-alpha tocopherol, and other ingredients. The 0.5 mg and 2 mg dosage strengths also contain yellow iron (ferric) oxide and brown iron (ferric) oxide.

Sirolimus inhibits T-lymphocyte activation and proliferation that occurs in response to antigenic and cytokine (Interleukin [IL]-2, IL-4, and IL-15) stimulation by a mechanism that is distinct from that of other immunosuppressants. Sirolimus also inhibits antibody production. In cells, Sirolimus binds to the immunophilin, FK Binding Protein-12 (FKBP-12), to generate an immunosuppressive complex. The Sirolimus: FKBP-12 complex has no effect on calcineurin activity. This complex binds to and inhibits the activation of the mammalian Target Of Rapamycin (mTOR), a key regulatory kinase. This inhibition suppresses cytokine-driven T-cell proliferation, inhibiting the progression from the G1 to the S phase of the cell cycle.

Studies in experimental models show that Sirolimus prolongs allograft (kidney, heart, skin, islet, small bowel, pancreatico-duodenal and bone marrow) survival in mice, rats, pigs, and/or primates. Sirolimus reverses acute rejection of heart and kidney allografts in rats and prolongs the graft survival in presensitized rats. In some studies, the immunosuppressive effect of Sirolimus lasts up to 6 months after discontinuation of therapy. This tolerization effect is alloantigen-specific.

In rodent models of autoimmune disease, Sirolimus suppresses immune-mediated events associated with systemic lupus erythematosus, collagen-induced arthritis, autoimmune type I diabetes, autoimmune myocarditis, experimental allergic encephalomyelitis, graft-versus-host disease, and autoimmune uveoretinitis.

The use of topical Sirolimus in the treatment of psoriasis was published by AD Ormerod et.al in Br J Dermatol, 152(4):758-64 (2005). It was a study published in 2005 that found a significant reduction in the clinical score but measurements of plaque thickness and erythema did not show significant improvement. It was concluded that topically applied Sirolimus penetrated normal skin and may have some antipsoriatic and immunosuppressive activity.

Numerous references such as B Madke, Indian Dermatol Online J., 4(1): 54-57 (2013) are available to extemporaneous formulations used to treat facial angiofibromas associated with tuberous sclerosis. Various investigators have shown that topically applied Sirolimus causes regression of facial angiofibromas with acceptable cosmetic results. The tumors return when topical use is discontinued. No detectable systemic absorption of Sirolimus (>1.0 ng/mL) was found. A substantial placebo controlled phase 2 study is currently ongoing.

A study of the efficacy of topical Sirolimus vs. topical betamethasone in chronic erosive oral lichen planus was terminated due to per protocol interim analyses of 76 patients and enrollment difficulties. A clinical trial to test whether topical Sirolimus is an effective treatment for the fibrofolliculomas found in Birt-Hogg-Dubé Syndrome (a rare genetic disorder) has been conducted and the results are currently under evaluation. There is a small open label study of the use of topical Sirolimus in Early Stage Cutaneous T-cell Lymphoma (CTCL) which has not yet begun to recruit subjects. A small (5 subject) study of subconjunctival Sirolimus for the treatment of autoimmune active anterior uveitis showed subconjunctival Sirolimus to be well tolerated and showed promise as a treatment for active inflammation in patients with chronic anterior uveitis. There are some promising publications on the topical delivery of Sirolimus to the eye for the treatment of retinal diseases. DE-109, an intravitreal formulation of Sirolimus, has been studied in dry eye syndrome, age-related macular degeneration, and diabetic macular edema. DE-109 is currently being evaluated in a phase 3 trial in patients with active noninfectious uveitis (www.clinicaltrials.gov).

Topical application of 1% Sirolimus can suppress the angiogenesis pathways and, therefore, reduce the regeneration and revascularization of photocoagulated blood vessels as described in Lasers Surg Med., 44(10):796-804 (2012).

Topical Sirolimus is being evaluated in Pachyonychia Congenita (a rare congenital disorder of the nails) in early phase trials. The topical formulation is being developed through an agreement with Pfizer and TransDerm, Inc to avoid the side effects found with oral Rapamycin in an earlier study.

The use of topical Sirolimus for treating inflammatory skin diseases (dermatitis, including psoriasis and lichen ruber planus) is described in CA2078379 patent application.

Following the administration of Rapamune Oral Solution, the mean times to peak concentration (tmax) of Sirolimus are approximately 1 hour and 2 hours in healthy subjects and renal transplant patients, respectively. The systemic availability of Sirolimus is low, and was estimated to be approximately 14% after the administration of Rapamune Oral Solution. In healthy subjects, the mean bioavailability of Sirolimus after administration of the tablet is approximately 27% higher relative to the solution. Sirolimus tablets are not bioequivalent to the solution; however, clinical equivalence has been demonstrated at the 2 mg dose level. Sirolimus concentrations, following the administration of Rapamune Oral Solution to stable renal transplant patients, are dose-proportional between 3 and 12 mg/m².

To minimize variability in Sirolimus concentrations, both Rapamune Oral Solution and Tablets should be taken consistently with or without food. In healthy subjects, a high-fat meal (861.8 kcal, 54.9% kcal from fat) increased the mean total exposure (AUC) of Sirolimus by 23 to 35%, compared with fasting. The effect of food on the mean Sirolimus Cₘₐₓ was inconsistent depending on the Rapamune dosage form evaluated.

The mean (± SD) blood-to-plasma ratio of Sirolimus was 36 ± 18 in stable renal allograft patients, indicating that Sirolimus is extensively partitioned into formed blood elements. The mean volume of distribution (Vss/F) of Sirolimus is 12 ± 8 L/kg. Sirolimus is extensively bound (approximately 92%) to human plasma proteins, mainly serum albumin (97%), α1-acid glycoprotein, and lipoproteins.

Sirolimus is a substrate for both CYP3A4 and P-gp. Sirolimus is extensively metabolized in the intestinal wall and liver and undergoes counter-transport from enterocytes of the small intestine into the gut lumen. Inhibitors of CYP3A4 and P-gp increase Sirolimus concentrations. Inducers of CYP3A4 and P-gp decrease Sirolimus concentrations. Sirolimus is extensively metabolized by O-demethylation and/or hydroxylation. Seven (7) major metabolites, including hydroxy, demethyl, and hydroxydemethyl, are identifiable in whole blood. Some of these metabolites are also detectable in plasma, fecal, and urine samples. Sirolimus is the major component in human whole blood and contributes to more than 90% of the immunosuppressive activity.

After a single dose of [¹⁴C] Sirolimus oral solution in healthy volunteers, the majority (91%) of radioactivity was recovered from the feces, and only a minor amount (2.2%) was excreted in urine. The mean ± SD terminal elimination half-life (t½) of Sirolimus after multiple dosing in stable renal transplant patients was estimated to be about 62 ± 16 hours.

Sirolimus was originally marketed as an oral solution . It requires cold storage and before administration it required mixing with water or orange juice. In order to increase the patient compliance, a tablet formulation was developed containing drug nanocrystals. The particle size reduction of the drug resulted in 23-27 % higher bioavailability compared to the oral solution. In spite of the achieved benefits, the oral bioavalability of the tablet formulation is still low (14%). To minimize variability in Sirolimus concentrations, both Rapamune oral solution and tablets should be taken consistently with or without food. Moreover, current production method is complicated and expensive.

WO2006094507 describes a pharmaceutical composition comprising sirolimus (rapamycin). The disclosed composition exhibits a releasing of sirolimus in a controlled manner so that the plasma levels stay within the narrow therapeutic window, that is, said composition has an extended release profile, where the peak concentration has been reduced. In other words, said composition has a modified release profile delivering significantly lower Cₘₐₓ. In contrast, our invention provides a novel composition having significantly improved release profile, immediate release and higher Cₘₐₓ. Additionally, the cited document discloses a method for the preparation of the described composition, which comprises the following steps: a) dissolving or dispersing sirolimus in a vehicle at a temperature of 50-80°C; b) adding the obtained mixture to a composition in powder or particulate form comprising excipients; and c) manufacturing the obtained powder into a composition. The disclosed method does not contain the step of the continuous flow mixing of two different solutions according to our invention.

EP1759724 discloses implantable medical devices, which may be coated with biocompatible materials containing one or more therapeutic drugs, such as sirolimus. The therapeutic drug is incorporated into a polymeric matrix or affixed directly to the system's components. The cited document also describes the preparation of ethanolic based Sirolimus solution containing TPGS, where said liquid formulations may be administered using catheter-based delivery systems. However, the use of said liquid formulation for oral administration and the step of the continuous flow mixing of two different solutions according to our invention are not disclosed.

EP1952807 discloses a composition comprising sirolimus in amorphous form, a fatty acid ester and a polymer, wherein the ester is glyceryl behenate, and the polymer is hydroxypropylcellulose (HPC), hydroxypropylmethylcellulose (HPMC) or polyvinylpyrrolidone (PVP). The cited document also relates to the preparation of the disclosed composition. The disclosed preparation method comprises the following steps: a) dissolving sirolimus, colloidal anhydrous silica and HPC in ethanol; b) dispersing the obtained solution in pregelatinized starch; c) granulation in a fluid-bed dryer; d) adding glyceryl behenate and microcrystalline cellulse; and e) tableting and coating. The disclosed method does not contain the step of the continuous flow mixing of two different solutions according to our invention.

US20130150397 discloses a dry, flowable and compressible Rapamycin composition comprising block copolymers of poly(ethylene oxide)-poly(propylene oxide) (Pluronics), for example, Pluronic F127. The cited document also described a preparation method of the disclosed composition: a) mixing the different copolymers with the Rapamycin in an organic solvent; and b) drying the obtained organic mixture. The disclosed Rapamycin composition does not comprise sodium lauryl sulfate. Additionally, the disclosed preparation method is not a continuous flow mixing process.

US2013039951 (& WO2013022201) discloses a process of preparing a formulation of a sirolimus derivative and a process for preparing a stable sirolimus-complex, comprising the steps of mixing a sirolimus derivative with water, an organic solvent, or a mixed solvent thereof; and contacting the obtained solution of the sirolimus derivative with a water-soluble carrier, to disperse the sirolimus derivative in the water-soluble carrier, into fluid bed granulator or high speed shearing mixer. The water-soluble carrier is one or more selected from the group consisting of polyvinyl pyrrolidone (PVP), hydroxypropyl cellulose (HPC), polyethylene glycol (PEG), saturated polyglycolised glycerides, hydroxypropyl cellulose, vinyl pyrrolidone-vinyl acetate copolymer.

In order to overcome the problems associated with prior conventional Sirolimus formulations and available drug delivery systems, novel complex formulation of Sirolimus or its salts and complexing agents and pharmaceutically acceptable excipients characterized by increased apparent solubility, instantaneous dissolution, increased permeability and enhanced biological performance including significantly improved exposure, earlier tₘₐₓ, higher Cₘₐₓ and higher trough concentrations at 24 hours which will allow the reduction of the dose was developed. Furthermore, the complex of the present invention possesses exceptional stability as a redispersed solution allowing the development of liquid based formulation for transdermal and other topical applications.

A variety of strategies have been used to attempt to overcome these issues, see for example US20100098770, WO2007091059, US20130150397, US20090068266, WO2010130982 WO2006101972, WO2007079560, EP2522338, US20100183728, US20080138405, US20130280336 US20130039951, US20090130210, WO2013022201 US20080176888 WO2008022557, US6565859, US6239102 WO2011135580, WO2006094507 WO2011128910, EP1781671, US20110009325, EP2575889, EP1273288, EP0650357, EP1670437 EP1871343, EP2480207, US20110076308, US20060251710, EP2402350 US20120022095, US20130225631, JP2005312967, WO2012142145, US20050025810, US20060263409, EP2402350, WO2006123226, US20050032680 and US20050239724.

### DESCRIPTION OF THE INVENTION

Disclosed herein is a stable complex comprising a) as active compound chosen from Sirolimus or its salts; b) polyvinylpyrrolidone as a complexing agent; and c) sodium lauryl sulfate as a pharmaceutically acceptable excipient. Said complex is obtained by continuous flow mixing process. Said complex is characterized in that it possesses the following property a), and optionally one of the following properties b) to i):
a) a particle size in the range between 50 nm and 600 nm, preferably 50 nm and 200 nm;
b) is instantaneously redispersable in physiological relevant media
c) is stable in solid form and in colloid solution and/or dispersion at least for 3 months;
d) has a PAMPA permeability of at least 2.0^{∗}10⁻⁶ cm/s, which does not decrease in time at least for 3 months;
e) has characteristic infrared (ATR) and or Raman absorption peaks/bands;
f) said complex has decreased fed/fasted effect;
g) said complex has significantly improved exposure, earlier tₘₐₓ, higher Cₘₐₓ and higher trough concentrations at 24 hours which will allow the reduction of the dose,
h) said complex has faster onset of action; and
i) said complex enables the possibility of the development of liquid based formulation for transdermal and other topical applications based on stability of the formula in liquid form.

The invention is a complex Sirolimus formula having increased solubility, instantaneous dissolution, increased permeability, decreased fed/fasted effect, higher AUC, Cₘₐₓ, faster onset of action and higher trough concentrations at 24 hours which will allow the reduction of the dose compared to unformulated compound and/or to the marketed drugs such as Rapamune described in the present invention. The present invention satisfies this need. Furthermore, the complex of the present invention possesses exceptional stability as a redispersed solution allowing the development of liquid based formulation for transdermal and other topical applications.

We have found that only the selected combinations of stabilizers and pharmaceutically acceptable excipients disclosed in the present invention result in a stable complex formulae having improved physicochemical characteristics and enhanced biological performance.

The expression Sirolimus is generally used for Sirolimus or its salts.

The complexing agent is chosen from polyvinylpyrrolidone (e.g Plasdone K-12, PVP 40, PVP K90, PVP 10).

The pharmaceutically acceptable excipient is sodium-lauryl-sulfate.

The complex has a controlled particle size in the range between 50 nm and 600 nm. In an embodiment, said particle size is between 50 nm and 200 nm.

In an embodiment, said complex further comprises one or more additional active agents.

In an embodiment, said additional active agent is chosen from agents useful for the prophylaxis of organ rejection in patients receiving renal transplants, for the treatment of psoriasis, facial angiofibromas associated with tuberous sclerosis, fibrofolliculomas found in Birt-Hogg-Dubé Syndrome, chronic erosive oral lichen planus, Early Stage Cutaneous T-cell Lymphoma, Treatment of Autoimmune Active Anterior Uveitis, dry eye syndrome, age-related macular degeneration, diabetic macular edema, noninfectious uveitis, telangiectasia, inflammatory skin diseases (dermatitis, including psoriasis and lichen ruber planus), Pachyonychia Congenita and for the suppression angiogenesis pathways.

In an embodiment, said complex possesses at least two of the properties described in a) - i).

In an embodiment, said complex possesses at least three of the properties described in a) - i).

In an embodiment, said complex has an increased dissolution rate.

Further disclosed herein is a stable complex comprising a) an active compound selected from the group of Sirolimus or its salt; b) polyvinylpyrrolidone as a complexing agent; and c) sodium-lauryl-sulfate as a pharmaceutically acceptable excipient; wherein said complex is obtained via a mixing process, according to claim 1.

The complex is obtained via a continuous flow mixing process.

In an embodiment, a complex comprises a complexing agent which is a polyvinylpyrrolidone and a pharmaceutically acceptable excipient which is sodium-lauryl-sulfate, in a total amount ranging from about 1.0 weight% to about 95.0 weight % based on the total weight of the complex.

In an embodiment, said complexing agent which is a polyvinylpyrrolidone and pharmaceutically acceptable excipient which is sodium-lauryl-sulfate comprise 50 weight% to about 95 weight% of the total weight of the complex.

Further disclosed herein is a process for the preparation of the complex, comprising the steps of mixing a solution of Sirolimus or its salt and polyvinylpyrrolidone as a complexing agent in a pharmaceutically acceptable solvent with an aqueous solution as an antisolvent containing sodium-lauryl-sulfate as a pharmaceutically acceptable excipient.

Said process is performed in a continuous flow instrument.

In an embodiment, said continuous flow instrument is a microfluidic flow instrument.

In an embodiment, said pharmaceutically acceptable solvent is chosen from methanol, ethanol, isopropanol, n-propanol, acetone, acetonitrile, dimethyl-sulfoxide, tetrahydrofuran, or combinations thereof.

In an embodiment, said pharmaceutically acceptable solvent is methanol.

In an embodiment, said pharmaceutically acceptable solvent and said aqueous solvent are miscible with each other.

In an embodiment, said aqueous solvent comprises 0.1 to 99.9% weight of the final solution.

In an embodiment, said aqueous solvent comprises 50 to 90% weight of the final solution.

In an embodiment, said aqueous solvent comprises 50 to 80% weight of the final solution.

In an embodiment, said aqueous solvent comprises 50 to 70% weight of the final solution.

In an embodiment, said aqueous solvent comprises 50 to 60% weight of the final solution.

In an embodiment, said aqueous solvent comprises 50 % weight of the final solution.

In an embodiment a pharmaceutical composition comprises the complex together with pharmaceutically acceptable carrier.

In an embodiment, said composition is suitable for oral, pulmonary, rectal, colonic, parenteral, intracisternal, intravaginal, intraperitoneal, ocular, otic, local, buccal, nasal, or topical administration.

In an embodiment, said composition is suitable for oral administration.

In an embodiment, said composition is suitable for topical/dermal administration.

In an embodiment, said complex is for use in the manufacture of a medicament for the prophylaxis of organ rejection in patients receiving renal transplants, for the treatment of psoriasis, facial angiofibromas associated with tuberous sclerosis, fibrofolliculomas found in Birt-Hogg-Dubé Syndrome, chronic erosive oral lichen planus, Early Stage Cutaneous T-cell Lymphoma, Autoimmune Active Anterior Uveitis, dry eye syndrome, age-related macular degeneration, diabetic macular edema, noninfectious uveitis, telangiectasia, inflammatory skin diseases (dermatitis, including psoriasis and lichen ruber planus), Pachyonychia Congenita and for the suppression angiogenesis pathways.

In an embodiment, said complex is used for the prophylaxis of organ rejection in patients receiving renal transplants, for the treatment of psoriasis, facial angiofibromas associated with tuberous sclerosis, fibrofolliculomas found in Birt-Hogg-Dubé Syndrome, chronic erosive oral lichen planus, Early Stage Cutaneous T-cell Lymphoma, Autoimmune Active Anterior Uveitis, dry eye syndrome, age-related macular degeneration, diabetic macular edema, noninfectious uveitis, telangiectasia, inflammatory skin diseases (dermatitis, including psoriasis and lichen ruber planus), Pachyonychia Congenita and for the suppression angiogenesis pathways.

In an embodiment, a method for reducing the therapeutically effective dosage of Sirolimus compared to orally available solid dosage forms and dosage forms for topical/dermal treatment comprises oral or topical/dermal administration of a pharmaceutical composition as described herein.

Further disclosed herein is a stable complex comprising
a. 10 - 40% by weight of Sirolimus or its salt;
b. 20 - 80% by weight of a polyvinylpyrrolidone; and
c. 5 - 50 % by weight of sodium-lauryl sulfate.
wherein said complex has a controlled particle size in the range between 50 nm and 600 nm; and wherein said complex is not obtained via a milling process or by high pressure homogenization process, encapsulation process and solid dispersion process, but it is obtained by continuous flow mixing process.

In an embodiment, said particle size is between 50 nm and 200 nm.

In an embodiment, said complex shows reduced fed/fasted effect based on *in vivo* studies.

In an embodiment, said complex shows significantly improved exposure, earlier tₘₐₓ, higher Cₘₐₓ and higher trough concentrations at 24 hours which will allow the reduction of the dose.

In an embodiment, said complex has a faster onset of action compared to the existing oral formulations.

In an embodiment, said complex is instantaneously redispersable in physiological relevant media.

In an embodiment, said complex is stable in solid form and in colloid solution and/or dispersion.

In an embodiment, said complex has a PAMPA permeability of at least 2.0^{∗}10⁻⁶ cm/s, which does not decrease in time at least for 3 months.

In an embodiment, said complex a) has characteristic infrared (ATR) and or Raman absorption peaks/bands.

The complexing agents and pharmaceutically acceptable excipients of the Sirolimus complex formulae of the invention are selected from the group of pharmaceutically acceptable nonionic, anionic, cationic, ionic polymers, surfactants and other types of excipients. The complexing agents themselves or together with the pharmaceutically accepted excipients have the function to form a complex structure with an active pharmaceutical ingredient through non-covalent secondary interactions. The secondary interactions can form through electrostatic interactions such as ionic interactions, H-bonding, dipole-dipole interactions, dipole-induced dipole interactions, London dispersion forces, π-π interactions, and hydrophobic interactions. The complexing agents, pharmaceutically accepted excipients and active ingredients are selected from the group of complexing agents, pharmaceutically accepted excipients and active ingredients which are able to form such complex structures through non-covalent secondary interactions.

In some embodiments, the compositions may additionally include one or more pharmaceutically acceptable excipients, auxiliary materials, carriers, active agents or combinations thereof. In some embodiments, active agents may include agents useful for the treatment hormone receptor positive metastatic breast cancer in postmenopausal women with disease progression following anti-estrogen therapy.

Another aspect of the invention is the complex formulae of the Sirolimus with complexing agents and pharmaceutically acceptable excipients in which the complexing agents and pharmaceutically acceptable excipients preferably are associated or interacted with the Sirolimus especially as the results of the mixing process, namely continuous flow mixing process. In some embodiment, the structure of the complex Sirolimus formula is different from the core-shell type milled particle, precipitated encapsulated particles, micelles and solid dispersions.

The pharmaceutical composition of the invention can be formulated: (a) for administration selected from the group consisting of oral, pulmonary, rectal, colonic, parenteral, intracisternal, intravaginal, intraperitoneal, ocular, otic, local, buccal, nasal, and topical administration; (b) into a dosage form selected from the group consisting of liquid dispersions, gels, aerosols, ointments, creams, lyophilized formulations, tablets, capsules; (c) into a dosage form selected from the group consisting of controlled release formulations, fast melt formulations, delayed release formulations, extended release formulations, pulsatile release formulations, and mixed immediate release and controlled release formulations; or (d) any combination of (a), (b), and (c).

The compositions can be formulated by adding different types of excipients for oral administration in solid, liquid, local (powders, ointments or drops), or topical administration, and the like.

The compositions can be formulated by adding different types of pharmaceutically acceptable excipients for oral administration in solid, liquid, local (powders, ointments or drops), or topical administration, and the like.

Preferred dosage forms of the invention are solid dosage forms and topical dosage forms, although any pharmaceutically acceptable dosage form can be utilized.

Solid dosage forms for oral administration include, but are not limited to, capsules, tablets, pills, powders, and granules. In such solid dosage forms, the active agent is admixed with at least one of the following excipients: (a) one or more inert excipients (or carriers), such as sodium citrate or dicalcium phosphate; (b) fillers or extenders, such as starches, lactose, sucrose, glucose, mannitol, microcrystalline cellulose and silicic acid; (c) binders, such as cellulose derivatives, alginates, gelatin, polyvinylpyrrolidone, sucrose and acacia; (d) humectants, such as glycerol; (e) disintegrating agents, such as crospovidon, sodium starch glycolate, effervescent compositions, croscarmellose sodium,, calcium carbonate, potato or tapioca starch, alginic acid, certain complex silicates and sodium carbonate; (f) solution retarders, such as acrylates, cellulose derivatives, paraffin; (g) absorption accelerators, such as quaternary ammonium compounds; (h) wetting agents, such as polysorbates, cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Besides such inert diluents, the composition can also include adjuvants, such as wetting agents, emulsifying and suspending agents, sweetening, flavoring, and perfuming agents.

Topical dosage forms include, but are not limited to powders, aerosols, plasters, lotions, liniments, solutions, emulsions, suspensions, ointments, creams, pastes, gels, jellies, suppositories. Compositions suitable for the topical, including transdermal, administration may comprise pharmaceutically acceptable topical ingredients, such as hydrophobic vehicle, water-miscible vehicle, co-solvent, structural matrix former, suspending, jelling, or viscosity inducing agents, water-in-oil (w/o) emulsifier, preservative, chelating agents, citric acid, edetic acid, sequestering antioxidant. Examples of pharmaceutically acceptable topical ingredients include *hydrocarbons* such as liquid petrolatum (mineral oil, liquid paraffin, paraffin oil), white petrolatum (petroleum jelly, Vaseline), yellow petrolatum (petroleum jelly), squalane (perhydrosqualene, spinacane), paraffin (paraffin wax, hard paraffin), microcrystalline wax, ceresin (mineral wax, purified ozokerite); *silicones* such as liquid polydimethylsiloxanes (dimethicone, silastic, medical grade silicone oil), fumed silica (cab-O-sil), bentonite (colloidal aluminum silicate), veegum (colloidal magnesium aluminum silicate); *alcohols* such as lauryl alcohols (1-dodecanol, dodecyl alcohols), myristyl alcohols (tetradecanol, tetradecyl alcohols), cetyl alcohols (hexadecanol, ethal, palmityl alcohols), stearyl alcohols (stenol, cetosteryl alcohols), oleyl alcohols (ocenol); *sterols and sterol esters* such as lanolin (hydrous wool fat, lanum), anhydrous lanolin (wool fat, anhydrous lanum, agnin), semi synthetic lanolin's; *carboxylic acids* such as lauric acid, myristic acid, palmitic acid, stearic acid, oleic acid; *esters and polyesters* such as cholesterol esters (stearate), ethylene glycol monoesters, propylene glycol monoesters, glyceryl monoesters, glyceryl monostearate, sorbitol monoesters, sorbitan monoesters, sorbitol diesters, sorbitan polyesters (spans, arlacels), glyceryl tristearate, lard, almond oil, corn oil, castor oil, cottonseed oil, olive oil, soybean oil, hydrogenated oils, sulfated oils, isopropyl myristate, isopropyl palmitate, polyoxyethylene sorbitan monoesters (stearate-tweens), polyoxy ethylene sorbitan polyesters (tweens), ethers and polyethers such as polyethylene glycol monocetyl ether (cetomacrogol 1000), polyethylene-polypropylene glycols (pluronics), polyoxy ethylene esters (stearate-polyethylene glycol monoesters, Myrj); bees wax, white bees wax (bleached bees wax), carnauba wax; *polyols and polyglycols* such as propylene glycol (1,2-propanediol), glycerin (glycerol), liquid polyethylene glycol, solid polyethylene glycol (hard macrogol, carbowax), 1,2, phenols-hexanetriol, sorbitol solution; *polycarboxylates, polysulfates and polysaccharides* such as agar, alginates, carragen, acacia, tragacanth, methylcellulose, carboxy methylcellulose, hydroxy ethyl cellulose, carboxy vinyl polymer, gelatin, pectin, xanthan, polyacrylic acid; *antioxidants* such as a-Tocopherol, ascorbic acid, ascorbyl palmitate, butylated hydroxyanisole, sodium ascorbate, sodium metabisulfite; buffers citric acid and salts, phosphoric acid and salts, H₃PO₄ / NaH₂PO₄, glycine, acetic acid, triethanolamine, boric acid, Ethylenediaminetetraacetic acid (Versene, EDTA); *humectant* such as glycerin (glycerol), propylene glycol (E 1520), glyceryl triacetate (E1518), sorbitol (E420), xylitol and maltitol (E965), polydextrose (E1200), quillaia (E999), lactic acid, urea, lithium chloride, *others* such as ethanolamin, riethanolamin, sodium lauryl sulfate, borax (sodium borate); presevatives such as benzalkonium chloride, benzoic acid, benzyl alcohol, bronopol, chlorhexidine, chlorocresol, imidazolidinyl urea, paraben esters, phenol, phenoxyethanol, potassium sorbate, sorbic acid and the like.

Advantages of the complex Sirolimus formulae of the invention include, but are not limited to (1) long term physical and chemical stability in solid form and in colloid solution and/or dispersion, (2) instantaneous redispersibility of the solid form, (3) stability in colloid solution or dispersion in the therapeutic time window, (4) increased solubility compared to the conventional Sirolimus formulations, (5) increased permeability, (6) increased Cₘₐₓ, AUC and earlier tₘₐₓ compared to the conventional Sirolimus formulations, (7) decreased fed/fasted effect, increased trough concentrations at 24 hours which will allow the reduction of the dose (8) and (9) good processability.

Beneficial features of the present invention are as follows: the good/instantaneous redispersibility of solid complex formulae of Sirolimus in water, biologically relevant media, e.g.; physiological saline solution, pH=2.5 HCl solution, FeSSIF and FaSSIF media and gastro intestinal fluids and long term stability in colloid solutions and/or dispersion in the therapeutic time window.

One of the preferred characteristics of the complex Sirolimus formulae of the present invention is their increased apparent solubility and permeability. In some embodiments, the permeability of the complex Sirolimus formulae is at least 2.0^{∗}10⁻⁶ cm/s and maintains it for at least 3 months.

Another preferred characteristic of the complex Sirolimus formulae of the present invention relates to the enhanced pharmacokinetic performance including significantly improved exposure, earlier tₘₐₓ, higher Cₘₐₓ and higher trough concentrations at 24 hours which will allow the reduction of the dose. Furthermore, the complex of the present invention possesses exceptional stability as a redispersed solution allowing the development of liquid based formulation for transdermal and other topical applications.

### BRIEF DESCRIPTION OF THE DRAWINGS

**Figure 1**. shows the complexing agent screening for formula selection in order to select the formulae having instantaneous redispersibility
**Figure 2****.** shows Comparative PAMPA assays of the unformulated compound, complex Sirolimus formula, marketed oral solution and marketed tablet form dispersed in different media
**Figure 3****.** shows PAMPA permeability of solid complex Sirolimus formula stored at 4°C, room temperature or 40 °C 75% relative humidity right after redispersion and at different time points (1, 7, 14, 34 and 95 days after the production)
**Figure 4****.** shows PAMPA permeability of colloid solution of complex Sirolimus formula stored at 4°C, room temperature or 40 °C 75% relative humidity and at different time points (1, 7, 14, 34 and 95 days after the production)
**Figure 5****.** shows plasma concentration of Sirolimus following the oral administration of the marketed drug (Rapamune tablet) and Sirolimus complex to rats under the fasting conditions (n=4, dose: 1.0 mg/kg)
**Figure 6****.** shows pharmacokinetic parameters following the oral administration of the marketed drug (Rapamume tablet) and the Sirolimus complex to rats under the fasting conditions (n=4, dose: 1.0 mg/kg)
**Figure 7**. shows dissolution test of capsule containing complex Sirolimus

### EXAMPLES

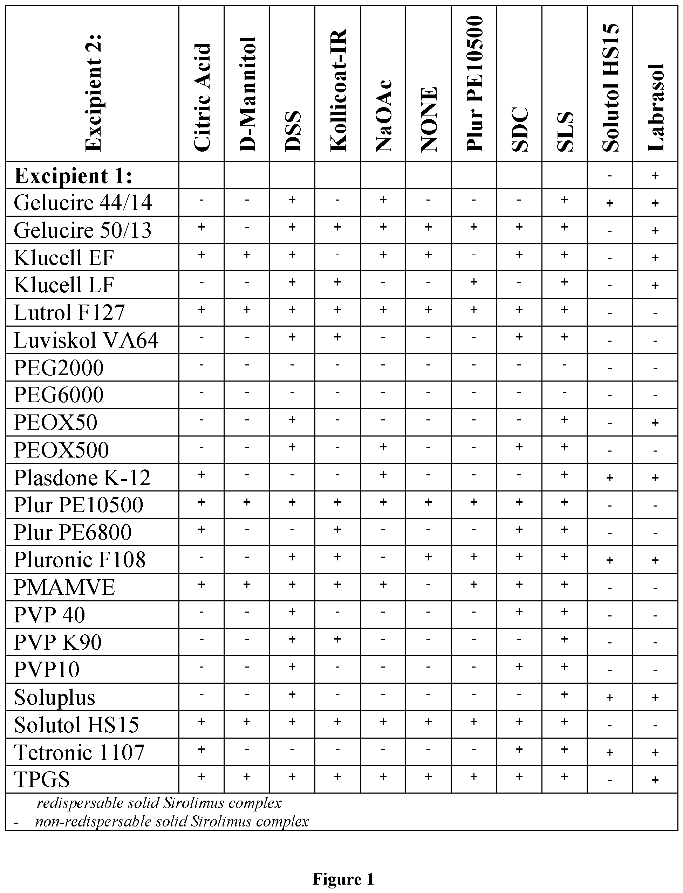

Several pharmaceutically accepted complexing agents and pharmaceutically accepted excipients and their combinations were tested in order to select the formulae having instantaneous redispersibility as shown in Figure 1. One of the examples that displayed an acceptable level of redispersibility was selected for further analysis.

Polyvinylpyrrolidone as complexing agent and sodium-lauryl sulfate as pharmaceutically accepted excipient were selected to form complex Sirolimus formulation having improved material characteristics.

The ratio of the selected complexing agent and pharmaceutically accepted excipient (polyvinylpyrrolidone and sodium-lauryl sulfate) was optimized making some slight differences in the preparation process to modify some characteristics of the product.

Colloid solution of Sirolimus complex formula of the present invention was prepared by continuous flow precipitation in a flow instrument. As a starting solution, 100 mg Sirolimus and 300 mg polyvinylpyrrolidone (PVP K90) dissolved in 100 mL methanol was used. The prepared solution was passed into the instrument with 5 mL/min flow rate. Meanwhile, antisolvent containing 250 mg sodium-lauryl sulfate in 500 mL water was passed into the instrument with 20 mL/min flow rate, where Sirolimus was precipitated to form complex Sirolimus composition. The colloid solution of the complex Sirolimus is continuously produced at atmospheric pressure. The produced colloid solution was frozen on dry-ice and then it was lyophilized using Scanvac CoolSafe 110-8 freeze drier equipped with -110°C ice condenser, with a Vacuubrand RZ6 vacuum pump.

In order to make the production process industrially feasible, process intensification was performed by increasing the concentrations of the starting solution. A colloid solution of Sirolimus complex formula of the present invention was prepared by continuous flow mixing in a flow instrument using the intensified process parameters. As a starting solution, 1000 mg Sirolimus and 3000 mg polyvinylpyrrolidone (PVP K90) dissolved in 100 mL methanol was used. The prepared solution was passed into the instrument with 10 mL/min flow rate. Meanwhile, aqueous solvent containing 2500 mg sodium-lauryl sulfate in 500 mL water was passed into the instrument with 40 mL/min flow rate, where Sirolimus formed complex Sirolimus composition.

### Comparative in vitro PAMPA assays

PAMPA permeability measurements were performed as described by M. Kansi et al. (Journal of medicinal chemistry, 41, (1998) pp 1007) with modifications based on S. Bendels et al (Pharmaceutical research, 23 (2006) pp 2525). Sample containing the reference compound or the marketed drug were a suspension of crystals visible by the naked eye, while samples of the novel complex were opalescent colloid solutions. Permeability was measured in a 96-well plate assay across an artificial membrane composed of dodecane with 20% soy lecithin supported by a PVDF membrane (Millipore, USA). The receiver compartment was phosphate buffered saline (pH 7.0) supplemented with 1% sodium dodecyl sulfate. The assay was performed at room temperature; incubation time was 1-24 hours. The concentration in the receiver compartment was determined by UV-VIS spectrophotometry (Thermo Scientific Genesys S10).

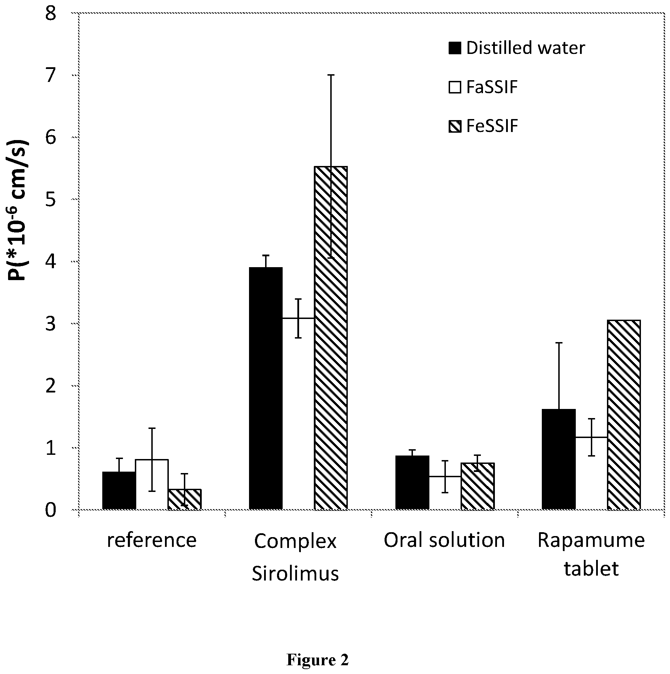

PAMPA permeability of complex Sirolimus formula of the present invention and the marketed drug was in the 2-6^{∗}10⁻⁶ cm/s range, while marketed Rapamune tablet exhibited at least 50% lower permeability when dispersed in water or in biorelevant media. The oral solution and the reference compound exhibited lower than 1^{∗}10⁻⁶ cm/s permeability in all dispersing media tested (Figure 2).

### Stability of the complex Sirolimus

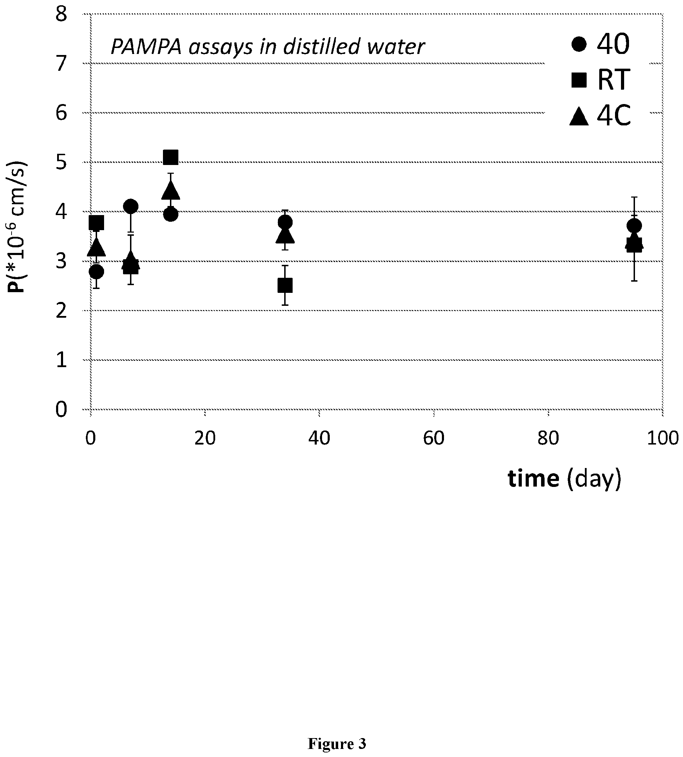

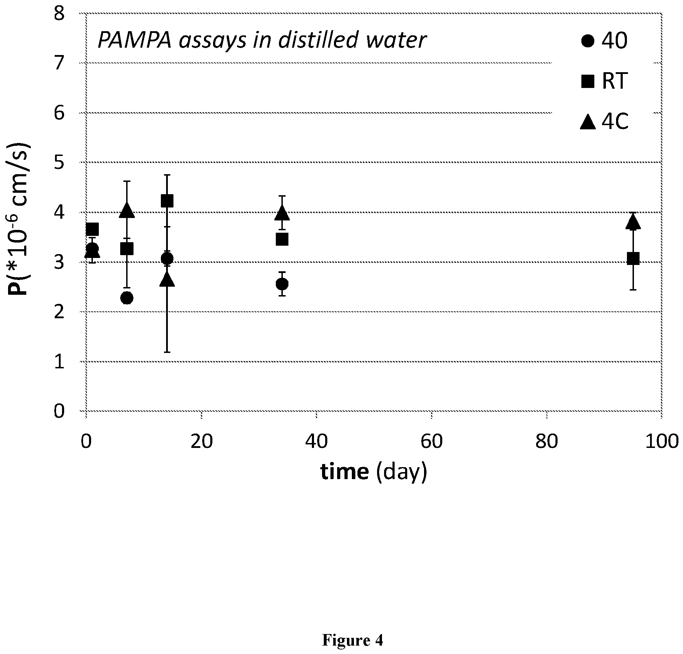

The solid forms and the redispersed colloid solutions were subjected to stability tests at 4°C, room temperature or 40°C/75% relative humidity for up to three months. The solids redispersed at the indicated time points exhibited no change in PAMPA permeability under any of the storage conditions (Figure 3). The redispersed colloids did not show significant change in PAMPA permeability for up to a month under all conditions, while no change was observed at 4°C or room temperature for up to 3 months (Figure 4).

### In-vivo pharmacokinetics

### In-vivo PK test in small animals

Male Wistar rats (250-270 g) were treated orally with either the test article or the reference article (1.0 mg/kg) one time via esophageal gavage technique following overnight fasting. Whole blood was sampled from the saphenous vein into heparinized tubes (approx. 0.2 mL to each tube) before and at 0.5, 1, 2, 3, 4, 6, 8, 24, and 48 hours after the oral administration of the test or reference articles. Whole blood samples were stored at minus 20°C until analysis. Sirolimus concentrations were determined from whole blood samples by an LC-MS/MS technique.

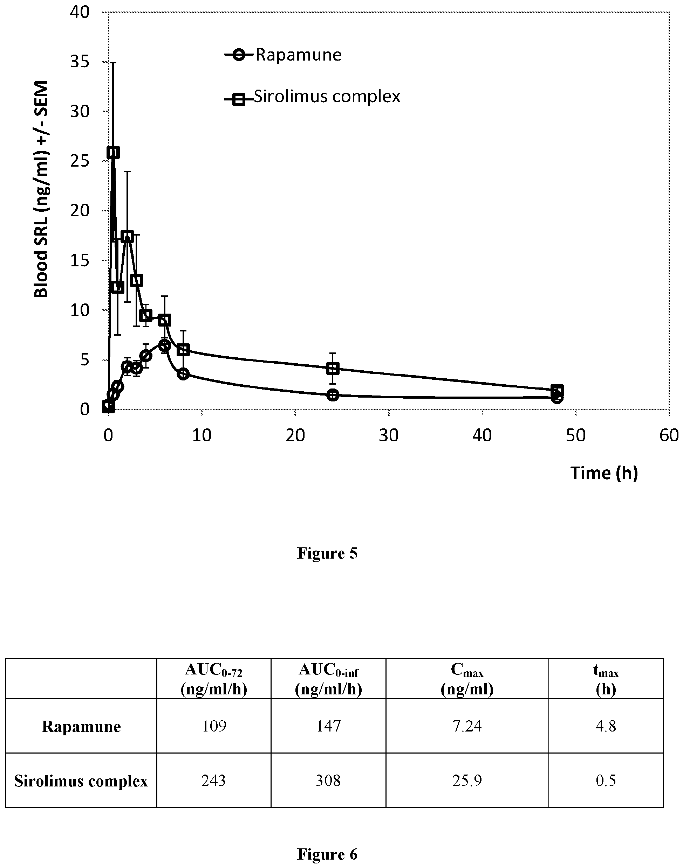

Pharmacokinetic data were expressed as measured values and as normalized values. Measured values were normalized to the extraction recovery of the standard solution from whole blood. Extraction recovery of the standard solution from whole blood varied between 94.0 and 114.7%. Following oral administration of Sirolimus as Sirolimus complex formula, tₘₐₓ was significantly decreased whilst Cₘₐₓ and AUC values increased when compared to the marketed tablet form, Rapamune. The trough concentrations measured as blood Sirolimus levels at 24 hours were also increased over 2-fold when compared to Rapamune (Figure 5-6).

### Pharmaceutical development

### Production of intermediate drug product of complex Sirolimus formula

Intermediate drug product was produced by mixing solid complex Sirolimus formula and commercially available excipient, Cellactose® 80 composing of 75% wt% lactose monohydrate and 25 wt% microcrystalline cellulose in the bracketing range of 0.5 and 2.0 mg strengths. For the production of intermediate drug product with 0.5 mg strength 9.23% wt% solid complex Sirolimus formula and 89.77% wt% Cellactose® 80 were mixed, while for 2.0 mg strength 1.67% wt% solid complex Sirolimus formula and 97.33% wt% Cellactose® 80 were mixed, respectively. Both powder mixtures were lubricated with 1% wt% magnesium stearate and filled into "1" size hard gelatin capsules. The powder mixture showed good flowability (USP <1174> and Ph.Eur. 2.9.16).

### In-vitro dissolution tests

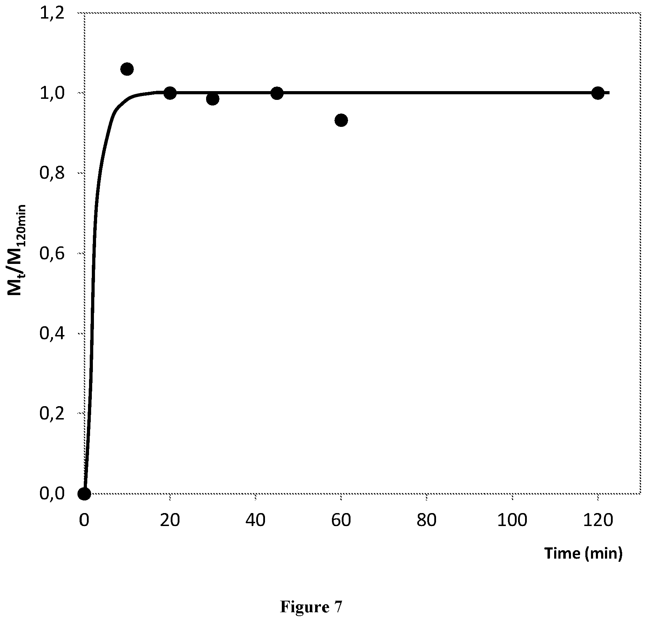

Dissolution test was performed by paddle method (Vankel VK7000) at 120 rpm and 37°C. Dissolution media was distilled water with 0.4% SLS (recommended by FDA). The concentration of Sirolimus was detected by UV-VIS spectrophotometry after filtration with a 1 µm pore size syringe filter at 279 nm wavelength. Complete dissolution of complex Sirolimus from the intermediate drug product was observed within 10 minutes (Figure 7).

### Preparation of topical gel formulation of complex Sirolimus formula

A gel formula for topical administration was developed using the novel complex Sirolimus formula. The gel was prepared by the sequential addition of the following components under continuous stirring: 1.800 g glycerol, 2.250 g 1 mg/ml sorbic acid solution, 3.828 g distilled water, 0.045 g Carbopol CP980 (as solid), 0.180 g 1 M sodium hydroxide solution and 0.900 g of the novel complex Sirolimus formula redispersed in distilled water at 10 mg/g concentration for active content.

## Claims

1. A stable complex comprising a) as active compound selected from the group of Sirolimus or its salts; b) polyvinylpyrrolidone as a complexing agent; c) sodium-lauryl-sulfate as a pharmaceutically acceptable excipient, wherein said complex is obtained by continuous flow mixing process and has a particle size in the range between 50 nm and 600 nm, preferably 50 nm and 200 nm.

2. The complex according to claim 1, wherein said complex further comprises one or more additional active agents, preferable the additional active agent is selected from the group of agents useful for the prophylaxis of organ rejection in patients receiving renal transplants, for the treatment of psoriasis, facial angiofibromas associated with tuberous sclerosis, fibrofolliculomas found in Birt-Hogg-Dubé Syndrome, chronic erosive oral lichen planus, Early Stage Cutaneous T-cell Lymphoma, Autoimmune Active Anterior Uveitis, dry eye syndrome, age-related macular degeneration, diabetic macular edema, noninfectious uveitis, telangiectasia, inflammatory skin diseases (dermatitis, including psoriasis and lichen ruber planus), Pachyonychia Congenita and for the suppression angiogenesis pathways.

3. A stable complex according to any of Claims 1 to 2, wherein said complex obtained by continuous flow mixing process in a microfluidic flow instrument.

4. A complex according to any of Claims 1 to 3, comprising a complexing agent which is a polyvinylpyrrolidone and a pharmaceutically acceptable excipient which is sodium-lauryl-sulfate, in a total amount ranging from about 1.0 weight% to about 95.0 weight % based on the total weight of the complex.

5. A process for the preparation of the complex according to any of Claims 1 to 4, comprising the steps of continuous flow mixing of a solution of Sirolimus or its salt and polyvinylpyrrolidone as a complexing agent in a pharmaceutically acceptable solvent with an aqueous solution as an antisolvent containing sodium-lauryl-sulfate.

6. The process according to Claim 5, wherein said process is performed in a continuous flow instrument, preferable in microfluidic instrument.

7. The process according to Claims 5 to 6, wherein said pharmaceutically acceptable solvent is selected from the group of methanol, ethanol, isopropanol, n-propanol, acetone, acetonitrile, dimethyl-sulfoxide, tetrahydrofuran, and combinations thereof, preferable the solvent is methanol.

8. The process according to any of Claims 5 to 7, wherein the solvent and the aqueous solvent are miscible with each other and the aqueous solvent comprises 0.1 to 99.9% weight of the final solution.

9. A pharmaceutical composition comprising the complex according to any of Claims 1 to 4 together with pharmaceutically acceptable carrier.

10. A pharmaceutical composition according to Claim 9, wherein said composition is suitable for oral, pulmonary, rectal, colonic, parenteral, intracisternal, intravaginal, intraperitoneal, ocular, otic, local, buccal, nasal, or topical administration, preferable the composition is suitable for oral and topical administration.

11. A complex according to any of Claims 1 to 4 for use in the prophylaxis of organ rejection in patients receiving renal transplants, in the treatment of psoriasis, facial angiofibromas associated with tuberous sclerosis, fibrofolliculomas found in Birt-Hogg-Dubé Syndrome, chronic erosive oral lichen planus, Early Stage Cutaneous T-cell Lymphoma, Autoimmune Active Anterior Uveitis, dry eye syndrome, age-related macular degeneration, diabetic macular edema, noninfectious uveitis, telangiectasia, inflammatory skin diseases (dermatitis, including psoriasis and lichen ruber planus), Pachyonychia Congenita and in the suppression angiogenesis pathways.

12. A stable complex comprising
a) 10 - 40% by weight of Sirolimus or its salt;
b) 20 - 80% by weight of a polyvinylpyrrolidone; and
c) 5 - 50 % by weight of sodium-lauryl-sulfate
wherein said complex has a controlled particle size in the range between 50 nm and 600 nm, preferable the particle size is between 50 nm and 200 nm; and wherein said complex is obtained according to any of claims 5 to 8.

## Patentansprüche

1. Ein stabiler Komplex, enthaltend a) einen Wirkstoff ausgewählt aus der Gruppe von Sirolimus oder dessen Salze; b) Polyvinylpyrrolidon als Komplexbildner; c) Natriumlaurylsulfat als pharmazeutisch annehmbaren Hilfsstoff, wobei der Komplex durch ein kontinuierliches Durchflussmischverfahren hergestellt wird und eine Partikelgröße im Bereich von 50 nm - 600 nm, vorzugsweise von 50 nm - 200 nm aufweist.

2. Der Komplex nach Anspruch 1, wobei der Komplex weiterhin einen oder mehrere zusätzliche Wirkstoffe enthält, der zusätzliche Wirkstoff vorzugsweise aus der Gruppe von Stoffen ausgewählt ist, die verwendbar ist für die Prophylaxe von Organabstoßung in Patienten nach Nierentransplantation, für die Behandlung von Psoriasis, Gesichtsangiofibromen im Zusammenhang mit tuberöser Sklerose, Fibrofollikulomen im Birt-Hogg-Dubé-Syndrom, chronischem erosivem oralem Lichen planus, Haut-T-Zell-Lymphom im Frühstadium, autoimmuner aktiver Uveitis anterior, Syndrom des trockenen Auges, altersbedingter Makuladegeneration, diabetischem Makulaödem, nichtinfektiöser Uveitis, Teleangiektasie, entzündlichen Hauterkrankungen (Dermatitis, einschließlich Psoriasis und Lichen ruber planus), Pachyonychia Congenita und für die Suppression der Angiogenesewege.

3. Ein stabiler Komplex nach einem der Ansprüche 1-2, wobei der Komplex durch ein kontinuierliches Durchflussmischverfahren in einem Mikrofluid-Durchflussgerät gewonnen wird.

4. Der Komplex nach einem der Ansprüche 1-3, enthaltend einen Komplexbildner, der ein Polyvinylpyrrolidon ist, und einen pharmazeutisch annehmbaren Hilfsstoff, welcher Natriumlaurylsulfat ist, in einer in einer Gesamtmenge von 1,0 Gewichts% - 95,0 Gewichts% bezogen auf das Gesamtgewicht des Komplexes.

5. Verfahren zur Herstellung des Komplexes nach einem der Ansprüche 1-4, umfassend die Schritte des kontinuierlichen Durchflussmischens der Lösung von Sirolimus oder ihres Salzes und von Polyvinylpyrrolidon als Komplexbildner in einem pharmazeutisch annehmbaren Lösungsmittel mit einer wässrigen Lösung als Antisolvens, welches Natriumlaurylsulfat enthält.

6. Das Verfahren nach Anspruch 5, wobei das Verfahren in einem kontinuierlichen Durchflussgerät, vorzugsweise in einem Mikrofluidgerät durchgeführt wird.

7. Das Verfahren nach den Ansprüchen 5-6, wobei das pharmazeutisch annehmbare Lösungsmittel ausgewählt ist aus der Gruppe Methanol, Ethanol, Isopropanol, n-Propanol, Aceton, Acetonitril, Dimethylsulfoxid, Tetrahydrofuran und Kombinationen dieser, vorzugsweise ist das Lösungsmittel Methanol.

8. Das Verfahren nach einem der Ansprüche 6-8, wobei das Lösungsmittel und das wässrige Lösungsmittel miteinander mischbar sind und das wässrige Lösungsmittel 0,1-99,9 Gewichts% der Endlösung enthält.

9. Eine pharmazeutische Zusammensetzung enthaltend den Komplex nach einem der Ansprüche 1-4 zusammen mit einem pharmazeutisch annehmbaren Trägerstoff.

10. Die pharmazeutische Zusammensetzung nach Anspruch 9, wobei die Zusammensetzung geeignet ist für orale, pulmonale, rektale, enterale, parenterale, intrazysternale, intravaginale, intraperitoneale, okulare, otische, lokale, bukkale, nasale oder topikale Verabreichung, vorzugsweise für die orale und topikale Verabreichung.

11. Der Komplex nach einem der Ansprüche 1-4 für die Verwendung in der Prophylaxe von Organabstoßung in Patienten nach Nierentransplantation, in der Behandlung von Psoriasis, Gesichtsangiofibromen im Zusammenhang mit tuberöser Sklerose, Fibrofollikulomen im Birt-Hogg-Dubé-Syndrom, chronischem erosivem oralem Lichen planus, Haut-T-Zell-Lymphom im Frühstadium, autoimmuner aktiver Uveitis anterior, Syndrom des trockenen Auges, altersbedingter Makuladegeneration, diabetischem Makulaödem, nichtinfektiöser Uveitis, Teleangiektasie, entzündlichen Hauterkrankungen (Dermatitis, enschließlich Psoriasis und Lichen ruber planus), Pachyonychia Congenita und in der Unterdrückung von Angiogenesevorgängen Suppression der Angiogenesewege.

12. Ein stabiler Komplex enthaltend
a) 10-40 Gewichts% Sirolimus oder sein Salz;
b) 20-80 Gewichts% Polyvinylpyrrolidon; und
c) 5-50 Gewichts% Natriumlaurylsulfat,
wobei der Komplex eine kontrollierte Teilchengröße im Bereich von 50 nm - 600 nm hat, vorzugsweise von 50 nm - 200 nm; und wobei der Komplex nach einem der Ansprüche 5 bis 8 gewonnen wird.

## Revendications

1. Complexe stable comprenant a) en tant que composé actif choisi dans le groupe du Sirolimus ou de ses sels; b) polyvinylpyrrolidone en tant qu'agent complexant; c) lauryl sulfate de sodium en tant qu'excipient pharmaceutiquement acceptable, où ledit complexe est obtenu par un procédé de mélange à flux continu et présente une taille de particules dans la plage allant de 50 nm à 600 nm, de préférence de 50 nm à 200 nm.

2. Complexe selon la revendication 1, où ledit complexe comprend en outre un ou plusieurs agents actifs supplémentaires, de préférence l'agent actif supplémentaire est choisi dans le groupe d'agents utiles pour la prophylaxie du rejet d'organe chez les patients recevant des greffes rénaux, pour le traitement du psoriasis, des angiofibromes faciaux associés à la sclérose tubéreuse, des fibrofolliculomes retrouvés dans le syndrome de Birt-Hogg-Dubé, du lichen plan buccal érosif chronique, du lymphome cutané à cellules T à un stade précoce, de l'uvéite antérieure active autoimmune, du syndrome de l'oeil sec, de la dégénérescence maculaire liée à l'âge, de l'œdème maculaire diabétique, de l'uvéite non infectieuse, de la télangiectasie, des affections cutanées inflammatoires (dermatite, y compris psoriasis et lichen plan), de la pachyonychie congénitale et pour la suppression des voies de l'angiogenèse.

3. Complexe stable selon l'une quelconque des revendications 1 à 2, où ledit complexe est obtenu par un procédé de mélange à flux continu dans un instrument à flux microfluidique.

4. Complexe selon l'une quelconque des revendications 1 à 3, comprenant un agent complexant qui est une polyvinylpyrrolidone et un excipient pharmaceutiquement acceptable qui est le lauryl sulfate de sodium, en une quantité totale allant d'environ 1,0% en poids à environ 95,0% en poids, relative au poids total du complexe.

5. Procédé de préparation du complexe selon l'une quelconque des revendications 1 à 4, comprenant les étapes consistant à mélanger en continu une solution de Sirolimus ou de son sel et de polyvinylpyrrolidone en tant qu'agent complexant dans un solvant pharmaceutiquement acceptable avec une solution aqueuse en tant qu'antisolvant contenant du lauryl sulfate de sodium.

6. Procédé selon la revendication 5, où ledit procédé est effectué dans un instrument à flux continu, de préférence dans un instrument microfluidique.

7. Procédé selon les revendications 5 à 6, où ledit solvant pharmaceutiquement acceptable est choisi dans le groupe du méthanol, de l'éthanol, de l'isopropanol, du n-propanol, de l'acétone, de l'acétonitrile, du diméthylsulfoxyde, du tétrahydrofurane et leurs combinaisons, de préférence le solvant est le méthanol.

8. Procédé selon l'une quelconque des revendications 5 à 7, où le solvant et le solvant aqueux sont miscibles l'un à l'autre et le solvant aqueux comprend 0,1 à 99,9% en poids de la solution finale.

9. Composition pharmaceutique comprenant le complexe selon l'une quelconque des revendications 1 à 4, conjointement avec un véhicule pharmaceutiquement acceptable.

10. Composition pharmaceutique selon la revendication 9, où ladite composition est appropriée pour une administration orale, pulmonaire, rectale, colonique, parentérale, intracisternale, intravaginale, intrapéritonéale, oculaire, otique, locale, buccale, nasale ou topique, de préférence, la composition est appropriée pour une administration orale et topique.

11. Complexe selon l'une quelconque des revendications 1 à 4, pour utilisation dans la prophylaxie du rejet d'organe chez les patients recevant des greffes rénaux, dans le traitement du psoriasis, des angiofibromes faciaux associés à la sclérose tubéreuse, des fibrofolliculomes retrouvés dans le syndrome de Birt-Hogg-Dubé, du lichen plan buccal érosif chronique, du lymphome cutané à cellules T à un stade précoce, de l'uvéite antérieure active auto-immune, du syndrome de l'oeil sec, de la dégénérescence maculaire liée à l'âge, de l'œdème maculaire diabétique, de l'uvéite non infectieuse, de la télangiectasie, des affections cutanées inflammatoires (dermatite, y compris psoriasis et lichen plan), de la pachyonychie congénitale et dans la suppression des voies de l'angiogenèse.

12. Complexe stable comprenant
a) 10 à 40% en poids de Sirolimus ou de son sel;
b) 20 à 80% en poids d'une polyvinylpyrrolidone; et
c) 5 à 50% en poids de lauryl sulfate de sodium
où ledit complexe a une taille de particule contrôlée dans la plage allant de 50 nm à 600 nm, de préférence, la taille de particule est comprise entre 50 nm et 200 nm; et où ledit complexe est obtenu selon l'une quelconque des revendications 5 à 8.
